# EUROPEAN PATENT APPLICATION

(11) **EP 4 272 704 A1**
(43) Date of publication of application: **08.11.2023**
(21) Application number: 22315095.4
(22) Date of filing: 06.05.2022
(51) Int. Cl.: A61F 2/24, A61F 2/95

(54) **COMPRESSING/LOADING A CARDIOVASCULAR IMPLANT**

(71) Applicant: Epygon, 13100 Aix-en-Provence (FR)
(72) Inventor: PECCHIO, Daniele, 13100 Aix-en-Provence (FR); VALERIO, Lorenzo, 13100 Aix-en-Provence (FR); FERRARO, Mauro, 13100 Aix-en-Provence (FR)
(74) Representative: Hepp Wenger Ryffel AG

(57) **Abstract**

Apparatus (10) for compressing a cardiovascular implant (40), optionally a prosthetic heart valve, to a small size for transcatheter implantation, comprising:
a funnel (20) having an interior surface defining a tapered channel (21) configured for compressing the implant in response to longitudinal translation of the implant within the channel, the channel having an axis (A) extending from an entrance (21a) to an exit (21b); and
at least one, optionally a plurality, of longitudinal cantilevered elements (32; 32a, 32b) configured for insertion into the channel via the entrance for supporting the implant (40) from within during compression, the at least one cantilever element having a tip biased towards a position spaced from the axis of the channel, the tip being deflectable towards the axis of the channel to accommodate tapering of the channel. The cantilever element biases the implant (40) outwardly against at least a portion of the interior surface of the funnel as the implant translates longitudinally within the channel, to prevent inward kinking during compression of an implant having a noncircular profile.

## Description

The present -invention relates to the field of cardiovascular implants, more specifically to apparatus, use and methods for implant compression and/or loading into a delivery catheter for transcatheter implantation. For example, the implant may be a cardiac implant and/or a prosthetic valve implant (e.g. a prosthetic cardiac valve).

Diseased, damaged or malformed heart valves often need to be treated with heart valve replacements. This is the case, for example, with valve regurgitation where the damaged valve no longer closes efficiently. The replacement prosthetic cardiac valve will generally be placed by a surgeon or other qualified practitioner either during open-heart surgery or by means of a less invasive method such as a transcatheter intervention. A transcatheter implantation has the potential to be highly beneficial and more efficient, as it allows the delivery of the implant without use of a heart and lung bypass machine. In this case the practitioner inserts a catheter through an access point to introduce a valve implant to the delivery site in a compressed condition for implantation.

Although pre-compressed valve implants have been proposed, the most common technique is to compress the implant from its normal size shortly before an intervention, in order to reduce risk of damage to the implant by being held compressed for too long. The compression of the implant is commonly done by use of a crimper that reduces the circumference of the stent thus allowing the delivery through minimally invasive techniques.

However, compressing an implant, especially a valve implant, is a complicated task. Implants are delicate and can break, deform, tear or be damaged if crimped incorrectly. In some cases the implant may still be used but will require re-crimping, which in turn may lead to a fragile or operationally less efficient device. In other cases the damage caused by incorrect compression renders the implant unusable and a new one is required. A second implant may not be immediately available, and preparing the second implant causes delay. Using a second implant will inevitably increase the cost of the procedure. The difficulty of compressing an implant is further exacerbated if the implant has a noncircular design. A noncircular implant may experience higher and non-uniform stress concentrations during crimping, increasing the risk of deformation such as kinking, or other damage to the implant. The implant also has to be loaded on a delivery catheter in its compressed state, which can also be challenging when the implant is of a self-expanding type. The implant must be kept sterile, and manipulation of the implant and crimper must be done while wearing sterile gloves. This can affect the dexterity of the operators, and make handling more awkward.

Further considerations relate to the number of people, especially trained operators, needed to perform each crimping procedure, and to load the implant on the catheter. Often multiple operators are needed to perform necessary stepwise operations and manipulations. However, the more operators that need to be relied on, the greater the potential for contamination and accidents through human error.

It may be a non-limiting object to address and/or alleviate at least one of the above issues, and/or to enhance a transcatheter delivery system as described above.

### Summary of the invention:

Broadly speaking, one aspect of the present invention provides an apparatus for compressing a cardiovascular implant to a small size for transcatheter implantation. The apparatus may comprise a funnel having an interior surface defining a tapered channel configured for compressing the implant in response to longitudinal translation of the implant within the channel. The apparatus is configured for compressing to a round form an implant having a noncircular profile.

As used throughout, the term "noncircular profile" refers to an implant having (i) a generally a non-round form and/or (ii) one or more out-of-round features. For example, a non-round form may include a D-shape in cross-section (for example, of a tubular part of the implant, described later below). An out-of-round feature may include a locally protruding anchor, optionally used in combination with a D-shape cross section of a tubular part of the implant. The implant may have the non-circular profile in a non-compressed and/or pre-compressed configuration, and/or in an operative implanted configuration.

Also, as used throughout, the term "compressing" refers to reducing the size of the implant at least in a circumferential direction of the implant, whether or not the implant may increase or decrease in axial length. Depending on the design of the implant, the implant may become longer when compressed circumferentially. Nevertheless, by being compressed circumferentially, the implant can be implanted using a small-diameter catheter to deliver the implant in its compressed configuration.

Also, as used throughout, and in all aspects below, the cardiovascular implant may optionally be a cardiac implant, optionally a prosthetic heart valve. The prosthetic heart valve may optionally be a prosthetic atrioventricular valve such as a mitral valve, and/or optionally be a self-expanding prosthetic heart valve. Another cardiovascular implant specifically envisaged is an implantable filter for filtering blood.

Additionally or alternatively to the first aspect, a second aspect of the present invention provides an apparatus for compressing a cardiovascular implant to a small size for transcatheter implantation. The apparatus comprises a funnel having an interior surface defining a tapered channel configured for compressing an implant in response to longitudinal translation of the implant within the channel. The apparatus further comprises a biasing device for biasing the implant outwardly against at least a portion of the interior surface of the funnel as the implant translates longitudinally within the channel. Optionally, the biasing resists inward kinking during compression of an implant having a noncircular profile.

The biasing device can help avoid kinking of the implant during compression by biasing the implant against the interior surface of the channel, thus significantly reducing the risk of accidental deformation of the implant during the compression phase.

In some embodiments, the biasing device, may comprise at least one cantilevered biasing element that extends longitudinally with regard to the channel and is configured to be biased outwardly toward at least a portion of interior surface of the channel. Additionally or alternatively, the cantilevered elements may be configured to be deflectable and/or capable of flexing inwardly so as to accommodate the tapering of the channel.

In other words, the cantilevered element(s) can be configured to conform to the channel as the implant advances longitudinally within in the channel. The cantilevered elements serve to keep the implant at least partially flush against the interior surface of the funnel, thus resisting inward kinking of the implant whilst being compressed.

In some embodiments, the biasing device may comprise at least two cantilevered elements; said cantilevered elements may have the same length and/or different lengths.

Additionally or alternatively to any of the above, the biasing elements of the biasing device may optionally be fingers extending from a hub. The fingers may extend in a longitudinal manner from the hub.

Having at least two of cantilevered biasing elements/fingers serves to keep the implant at least partially flush against the interior surface of the funnel at multiple positions around its entire cross-section, thus increasing the resistance to unwanted inward kinking.

In some embodiments, the apparatus may comprise an actuator mechanism; operation (e.g. manual operation) of the actuator mechanism may generate longitudinal movement of the biasing device with respect to the funnel. Optionally the actuator mechanism may comprise a screw threaded element generating the longitudinal movement from rotation of a rotatable actuator member. The actuator mechanism can thereby serve to advance the biasing device longitudinally progressively.

In some embodiments, the apparatus may comprise a mover for advancing the implant within the channel, the mover being driven by the movement of the actuator. The mover may be attached or attachable, directly or indirectly, to the implant allowing the implant to move along the tapered channel as the mover is activated by the actuator mechanism. For example, the mover may be a pulling device coupled or coupleable, directly or indirectly, to the implant for pulling the implant through the funnel. In one form, the mover comprises a shaft configured to be coupled to a connector that is pre-attached to the implant, for example, via one or more tethers or arms pre-attached to the implant.

Here, operation (e.g. manual operation) of the actuator mechanism drives the mover, thus advancing the implant in the channel. This can provide controllable compression of the implant as it longitudinally advances within the channel.

In some embodiments, the apparatus may comprise an actuator mechanism configured to cause simultaneous longitudinal movement of the biasing device and the mover over at least a part of the range of movement of the biasing device and/or of the mover. Optionally the actuator mechanism may induce a longitudinal displacement of the biasing device in unison with the longitudinal displacement of the mover over at least a part of the range of movement of the biasing device and/or of the mover.

Simultaneous longitudinal movement of the biasing device and the mover can ensure that they advance in coordination with one another, which can be advantageous for controlled compression of the implant, and continuous support for implant from within as the implant is moved.

Additionally or alternatively to either aspect above, and additionally or alternatively to any of the features described above, a third aspect of the invention provides apparatus for compressing a cardiovascular implant to a small size for transcatheter implantation. The apparatus comprises a funnel having an interior surface defining a tapered channel configured for compressing the implant in response to longitudinal translation of the implant within the channel, the channel having an axis extending from an entrance to an exit. The apparatus further comprises at least one, optionally a plurality, of longitudinal cantilever elements configured for insertion into the channel via the entrance. The at least one cantilever element has a tip biased towards a position spaced from the axis of the channel, the cantilever element and/or the tip being resilient to allow flexing and/or deflection towards the axis of the channel as the cantilever element advances within the tapered channel.

The at least one cantilever element may be configured to bias an implant outwardly into contact with the interior surface of the funnel, optionally to obstruct kinking of an (e.g. noncircular) implant.

Additionally or alternatively, the at least one cantilever element may be configured to guide folding of a valve component of a prosthetic cardiac valve implant during compression of the prosthetic cardiac valve implant.

Where plural cantilever elements are provided, two or more of the elements may have generally the same length, and/or two or more of the elements may have generally different lengths. For example, different lengths may be appropriate for cooperating with a valve component of a prosthetic cardiac valve implant, for guiding folding of the valve component.

Additionally or alternatively to any of the above aspects, and optionally using any of the above-described features, a fourth aspect of the invention provides a method of compressing a cardiovascular implant to a smaller size for transcatheter delivery. The method may optionally use an apparatus according to any of the preceding aspects. The method comprises:
translating the implant longitudinally within a funnel having an interior surface defining a tapered channel, such that the tapered channel compresses the implant in response to the longitudinal translation; and
during at least a part of the translation step, supporting at least one zone of the implant from radially within to resist kinking of said zone during compression.

The supporting step may comprise using a biasing device to bias the implant outwardly against at least a portion of the interior surface of the funnel as the implant translates longitudinally within the channel. The biasing device may comprise at least one, optionally plural, longitudinal cantilevered elements.

Additionally or alternatively to any of the above aspects, and optionally using any of the above-described features, a fifth aspect of the invention provides a method of compressing a cardiovascular implant to a smaller size for transcatheter delivery. The method may optionally use an apparatus according to any of the first to third aspects. The method comprises:
translating the implant longitudinally within a funnel having an interior surface defining a tapered channel, such that the tapered channel compresses the implant in response to the longitudinal translation; and
during at least a part of the translation step, supporting at least one zone of the implant from radially within by at least one, optionally a plurality of, longitudinal cantilever elements.

The method of either aspect above may further comprise inserting the biasing device and/or at least one cantilever element into or via an end (e.g. an entrance end) of the funnel and/or channel.

The method of either aspect above may further comprise translating the biasing device and/or at least one cantilever element longitudinally within the channel. The step of translation may cause the biasing device and/or at least one cantilever element to deflect and/or flex towards an axis of the channel as the biasing device and/or at least one cantilever element translates along the channel.

The biasing device and/or at least one cantilever element may be translated simultaneously with translation of the implant, over at least a part of the range of movement of the implant, and optionally substantially in unison with the implant over at least a part of the range of movement of the implant. This can provide continuous support for the implant during compression.

The method of either aspect above may further comprise manually operating an actuator mechanism to translate the implant and to translate the biasing device and/or at least one cantilever element, optionally at least partly simultaneously and/or in unison, as explained above. The step of manually operating the actuation mechanism may comprise rotating a rotary actuation member of the actuation mechanism to drive translation of: the implant (e.g via a mover); and/or of the biasing device; and/or of the at least one cantilever element. The step of rotating the rotary actuation member may comprise rotating the rotary actuation member generally around a rotation axis generally coaxial with and/or parallel to a longitudinal axis of the funnel.

Additionally or alternatively to any aspect above, and additionally or alternatively to any of the features described above, a sixth aspect of the invention provides apparatus for compressing a cardiovascular implant to a small size for transcatheter implantation. The apparatus comprises a funnel having an interior surface defining a tapered channel configured for compressing the implant in response to longitudinal translation of the implant within the channel. The tapered channel comprises a cross-section that varies in shape and size from one end of the funnel to the other, and/or along at least a part of the length of the channel. The shape of the channel may change between a non-round channel shape and a round channel shape for compressing to a round form an implant having a noncircular profile.

In any of the above aspects, the tapered channel of the apparatus permits the passage of the implant from a deployed state to a compressed state. The changing of the channel shape from a non-round or an out-of-round cross-section to a generally round cross-section can assist the implant in adopting a generally round compressed form, typical for introduction using a catheter. There is often a need for a non-round implant to adopt a round form when compressed, to fit within the catheter, and for delivery in a patient.

The tapered channel of the apparatus may comprise a cross-section that changes progressively in size and shape over at least a part of the length of the channel, for compressing to a round form an implant having a non-round form and/or out-of-round features.

The progressive change in the cross-section of the channel progressively imparts a change in shape and size to the implant according to its longitudinal advancement. This allows a gradual adaptation of the implant structure avoiding unnecessary stress on the implant and reducing the risk of damage.

In some embodiments, a portion of the channel may have a D-shaped cross-section including a curved region (e.g. representing the round part of the "D") connected to a less-curved region (e.g. representing the flat part of the "D"). The less-curved region may optionally include a curvilinear region or a straight region. Optionally the D-shaped cross-section may have one or more peripheral protuberances. A D-shaped cross-section can provide a channel that is adapted to a type of implant having a D-shaped configuration in its expanded state, such as some types of mitral valve implant. An implant having a D-shape can thus be compressed with more control over the shape than were the D-shaped implant to be compressed using a purely round funnel channel, which is inherently less intimately adapted to the implant shape.

Additionally or alternatively, in some embodiments, the interior surface of the funnel defining, the tapered channel may comprise one or more longitudinal interior grooves. The grooves provide an advantage of being able to adapt the channel shape to implants with out-of-round features, such as protuberances extending outwardly from a tubular implant. The position of at least one groove may be generally aligned with the position of such a protuberance.

In some embodiments, at least one groove, optionally multiple grooves, optionally each groove, flares circumferentially at a first end of the groove. The flare may correspond to a gradual circumferential widening at the first end of the longitudinal groove. For example, the first end may correspond to the wide and/or entrance end of the funnel. In one example, the groove has sidewalls that flare away from each other at the first end of the groove. The flaring may be generally linear, or curvilinear, or generally curved (e.g. convexly or concavely). Circumferential flaring can help guide a protuberance of the implant correctly into the groove as the implant enters the channel, allowing correction of any slight rotational misalignment, and providing position tolerance without unnecessary circumferential widening of the groove along a majority of its length.

In some embodiments, the implant may have an anchoring system comprising multiple protuberances at different circumferential positions around the periphery of the implant. The funnel may have fewer grooves than the number of protuberances, such that not all of the protuberances are accommodated in a respective groove. For example, the funnel may have grooves corresponding only to protuberances that fold into the wall of the implant to be coincident with the implant wall. For example, such protuberances may fold into window frames provided in a tubular portion of the implant wall.

In some embodiments, at least one groove has a depth that changes along at least part of the length of the channel. Optionally the depth may change progressively along at least a part of the length of the channel. Optionally the depth of the groove may become shallower towards an exit of the channel. Such configurations of groove can be configured to compress or fold inwardly protuberances of the implant as the implant advances longitudinally within the channel.

Additionally or alternatively to any of the above aspects, and optionally using any of the above-described features, a seventh aspect of the invention provides a method of compressing a cardiovascular implant to a smaller size for transcatheter delivery. The method may optionally use an apparatus according to the sixth aspect, or any other aspect. The method comprises:
translating the implant longitudinally within a funnel having an interior surface defining a tapered channel, such that the tapered channel compresses the implant in response to the longitudinal translation,
wherein the step of translating comprises causing the implant to change its cross-section size and shape by virtue of the channel varying in size and shape along at least part of the funnel.

The step of translating may comprise translating the implant such that at least one anchoring system protuberance of the implant enters a longitudinal groove of the funnel. Optionally, the protuberance may enter the groove via a mouth of the groove that tapers circumferentially to gradually guide the protuberance into the groove. Additionally or alternatively, the step of translating may comprise translating the implant such that at least one other anchoring system protuberance of the implant does not enter a respective groove of the funnel. The number of grooves may be fewer than the number of protuberances. Additionally or alternatively to the above, the step of translating may comprise translating the implant from a region in which the interior channel has a generally noncircular cross-section shape, to a region in which the interior channel has a generally round cross-section shape. For example, the noncircular cross-section shape may be a D-shape including a curved region (e.g. representing the round part of the "D") connected to a less-curved region (e.g. representing the flat part of the "D"). The less-curved region may optionally include a curvilinear region or a straight region. Optionally the D-shaped cross-section may'have one or more peripheral protuberances. Additionally or alternatively to any aspect above, and additionally or alternatively to any of the features described above, a fifth aspect of the invention provides apparatus for compressing a cardiovascular implant to a small size for transcatheter implantation, comprising a funnel having an interior surface defining a tapered channel configured for compressing the implant in response to longitudinal translation of the implant within the channel, the channel having an axis extending from an entrance end to an exit end. The apparatus may further comprise first and second members that are translatable longitudinally with respect to the channel, and optionally at least partly with respect to each other. The apparatus may further comprise an actuator mechanism for effecting longitudinal movement of the first element from (and/or at, and/or via) the entrance end of the channel, and for effecting longitudinal movement of the second element from (and/or at, and/or via) the exit end of the channel.

The first member may, for example, correspond to a biasing device as described above. The second member may, for example, correspond to a mover as described above.

The actuator mechanism may be configured to move the first and second members simultaneously with each other, at least over a part of the range of movement of one or both of the members. Optionally, the actuator mechanism may be configured to move the first and second members in unison with each other, at least over a part of the range of movement of one or both of the members.

Such an actuator mechanism can provide a single actuator that is able to cause movement of multiple parts relative to the compression channel, and can facilitate operation of the device by, for example, a single operator.

Additionally or alternatively to any aspect above, and additionally or alternatively to any of the features described above, an eighth aspect of the invention provides a holder carrying (or for carrying) a cardiovascular implant in a packaged configuration of the implant, the implant being compressible to a compressed configuration for transcatheter implantation, optionally using a compressor apparatus as defined in any of the preceding aspects.

The holder at least partly circumscribes at least a portion of the cardiovascular implant, such that the implant sits at least partly within the holder. The holder comprises an engagement profile that is configured to cooperate with an anchor of the implant, to bend the anchor from a deployed configuration towards a non-deployed configuration when the implant is translated out of'the holder at least in a first direction of relative movement. For example, in the deployed configuration, the anchor may protrude laterally with respect to a tubular portion of the implant. In the non-deployed configuration, the anchor may no longer protrude laterally, and/or it may have a more streamlined shape at least laterally.

With such an arrangement, the holder can serve multiple purposes. It can firstly serve to carry the .implant in its packaged configuration prior to use, for example, in a sterile bottle or other packaging. Secondly, it can be used to assist compression of the implant to the compressed configuration, by bending an anchor of the implant towards a non-deployed configuration as the anchor is translated out of the holder. This bending operation then does not need to be performed separately by a compressor apparatus.

In some embodiments, the holder additionally can be used to transfer the implant from its packaging to a compressor apparatus for compressing the implant, optionally a compressor apparatus according to any of the preceding aspects. For example, the apparatus may comprise a funnel having an interior surface defining a tapered channel configured for compressing the implant in response to longitudinal translation of the implant within the channel. The holder may be positionable near or at an entrance of the compressor, to bend the anchor when the implant is translated from the holder and into the funnel.

In some embodiments, the engagement profile may bend the anchor through at least 90°, optionally at least 120°, optionally at least 150°, optionally at least 170°, optionally about 180°.

In some embodiments, the implant comprises a plurality of anchors, and the holder comprises a plurality of engagement profiles configured to bend at least some of the plurality of anchors.

Additionally or alternatively to any aspect above, and additionally or alternatively to any of the features described above, a ninth aspect of the invention provides use of a holder for a cardiovascular implant, the cardiac implant being compressible from an expanded configuration to a compressed configuration for transcatheter implantation, the cardiac implant having at least one anchor movable between a deployed shape in the expanded configuration of the implant, and a non-deployed shape in the compressed configuration of the implant, the holder having at least one engagement profile for contacting the at least one anchor of the implant. The use comprises:
(i) using the holder to at least partly circumscribe at least a portion of the cardiovascular implant in a packaged configuration prior to use, whereby the implant sits at least partly within the holder; and
(ii) using the holder to at least partly bend the anchor from the deployed shape towards the non-deployed shape, by contact between the engagement profile and the anchor, when the cardiac implant is translated out of the holder for compression to the compressed configuration, whereby the holder facilitates compression of the implant.

The use may further include using the holder to support the cardiac implant at an insertion position with respect to (for example, an entrance to) a compression apparatus comprising a compression funnel for compressing the implant by longitudinal translation of the implant out of the holder and through the funnel.

Additionally or alternatively to any aspect above, and additionally or alternatively to any of the features described above, a tenth aspect of the invention provides a process or method comprising:
providing a cardiovascular implant in a packaged configuration mounted to a holder, the implant having an anchor for interacting with cardiovascular tissue in use, and the holder at least partly circumscribing the implant such that the implant sits at least partly within the holder; and
translating the implant out of the holder, the step of translating comprising causing an engagement profile of the holder to interact with the anchor as the implant is translated out of the holder, to bend the anchor from a deployed shape to a non-deployed shape.

Additionally or alternatively to any aspect above, and additionally or alternatively to any of the features described above, an eleventh aspect of the invention provides apparatus comprising:
(i) a cardiovascular implant in a packaged configuration, the implant being compressible to a compressed configuration for transcatheter implantation; and
(ii) a puller to which the implant is coupled by at least one tether extending along multiple return runs and/or multiple pairs of runs between the puller and the implant in such a manner to obstruct lengthwise slippage of the tether with respect to the puller.

Each return run includes a pair of individual runs, for example, out and back, between the puller and the implant.

The puller may be used to pull the implant into and/or through a compressor device (for example, a funnel) by means of the tethers. Obstructing lengthwise slippage of the tether with respect to the puller, can provide stable control of the relative spacing from the puller to the implant, around the periphery of the of the implant and/or puller, without having to provide a separate tether for each run. Implant stability is important to prevent the implant from slipping off-centre and/or affecting the distribution of the pulling forces on the implant, either of which may be detrimental to crimping.

For example, the puller may comprise a hub comprising a plurality of apertures through which the tether passes. The tether may include an anchoring turn to anchor at least one end of a return run of the tether with respect to at least one aperture, thereby to obstruct lengthwise slippage.

In some embodiments, the puller comprises at least one component with at least one, optionally a plurality, of cutting gaps at which the at least one tether is presented to facilitate cutting of the at least one tether to release the implant from the puller. The gaps may optionally take the form of a slot in the puller surface. For example, the technician may insert a scalpel into the gap to cut the tether at that point. Providing one or more cutting gaps in the puller promotes cutting at a controlled position, furthest from the implant, and in which a technician is least likely to accidentally damage the implant with a scalpel. It also avoids the technician having to cut the runs at some other imprecise and riskier position closer to the implant.

The cutting gap may be arranged such that, by cutting the tether within the gap, the tether separates into segments. The segments may remain attached to the hub. Such an arrangement can avoid the risk of loose segments of tether material being left attached to the implant, where the material would then represent foreign debris that could cause problems to the patient if accidentally implanted.

In some embodiments, there may be eight return runs, and four cutting gaps. Cutting the tether portion in a cutting gap

In some embodiments, the tether may extend along a path comprising, in order:
a first set of at least one return run between the puller and the implant;
a segment extending across a cutting gap; and
a second set of at least one return run between the puller and the implant.

Optionally, the first set of at least one return run is anchored to the puller at a first end position distant, lengthwise along the tether, from said segment. Additionally or alternatively, the second set of at least one return run is anchored to the puller at a second end position distant, lengthwise along the tether, from said segment. Such arrangements enable the first end and/or the second end to remain anchored to the puller when the segment is severed at the cutting gap.

In some embodiments, the puller comprises a hub with a plurality of apertures through which the at least one tether extends, and a cover covering at least partly the hub.

The at least one tether may comprise a single tether defining all of the multiple runs between the puller and the implant, or the at least one tether may comprise multiple tethers, for example, each extending around a respective sector of the periphery of the puller and/or implant. Four tethers may each extend around a respective quarter (approximately) of the periphery.

In any of the above, the multiple tethers may be substantially independent of one another. Alternatively, the multiple tethers could be joined by one or more links or joins.

Additionally or alternatively to any aspect above, and additionally or alternatively to any of the features described above, a twelfth aspect of the invention provides a method or process comprising:
coupling a cardiovascular implant to a puller by at least one tether extending along multiple return runs and/or multiple pairs of runs between the puller and the implant, in such a manner to obstruct lengthwise slippage of the tether with respect to the puller; and
packaging the cardiovascular implant and the puller.

In particular, the method process may optionally any of the features described above for the eleventh aspect.

Additionally or alternatively to any aspect above, and additionally or alternatively to any of the features described above, a thirteenth aspect of the invention provides an expansion restrictor of (or for) a delivery catheter, for assisting loading of a self-expanding cardiovascular implant onto the delivery catheter.

The delivery catheter comprises one or more or all of: an accommodation region for accommodating the cardiovascular implant in a compressed configuration; and/or a sheath translatable longitudinally with respect to the accommodation region for selectively covering the implant to restraint the implant in the compressed configuration or selectively uncovering the implant to allow expansion for implantation; and/or an implant retainer in the accommodation region for mating engagement with at least one attachment element of the implant.

The expansion restrictor comprises a tubular member configured for fitting over at least a portion of the sheath, the tubular member comprising a bore for receiving the sheath and terminating at a mouth at one end, the expansion restrictor further comprising at least one projection adjacent to the mouth, and a manipulator for moving the projection, the projection projecting radially inwardly with respect to a surface of the bore and being movable by the manipulator in a circumferential direction with respect to the tubular member. The at least one projection may be a single projection, or multiple projections distributed in the circumferential direction (e.g. two diametrically opposed projections). References herein to "the projection" may apply to multiple projections, as appropriate.

Such an expansion restrictor can provide multiple functions during loading. Firstly, the projection can provide a locking function to temporarily lock an attachment element of the implant to the implant retainer until the sheath of the delivery catheter begins to advance over the implant and restrain the implant. The manipulator can be operated to move the projection circumferentially into a locking position. Secondly, the tubular member around the sheath can reinforce the sheath to reduce risk of the sheath buckling as the sheath is pushed to close the sheath over the implant.

In more detail, the expansion restrictor can be used to temporarily retain the attachment element of the implant in operative engagement with the implant retainer of the delivery catheter, and prevent disengagement even while not covered by the sheath. Prior to closing the sheath, the self-expanding property of the implant makes it otherwise difficult to retain the attachment element engaged with the implant retainer, particularly for a single person performing the loading, because the implant is self-biased to expand out of engagement with the implant retainer when the sheath is open. With the projection of the restrictor in a non-locking position, the attachment element can easily be engaged with the implant retainer, after which the projection is moved to a locking position. The expansion restrictor can overcome the self-expanding bias, and block the attachment element in operative engagement with the implant retainer. This provides stable and reliable engagement without further interaction by the user. The reinforcement provided by the tubular member reduces risk of buckling when the sheath is translated over the self-expanding implant. Translating the sheath closed can directly or indirectly transition the attachment element from being restrained by the projection, to being restrained by the sheath, and without detaching from the implant retainer. Such a technique can simplify the loading operation, without increasing the load burden on the sheath, and facilitate advantageous loading of the delivery catheter by a single person.

In some embodiments, the at least one projection projects radially inwardly to a position substantially in register with an inner surface of the sheath.

In some embodiments, the projection extends axially beyond the mouth, the projection defining, for example, a circumferential castellation.

In some embodiments, the manipulator comprises a ring carrying the projection. Movement of the ring in a circumferential direction with respect to the tube, moves the projection carried by the ring. Rotary movement is straightforward and unambiguous for the operator to perform.

In some embodiments, the diameter of the bore in the tubular member is substantially the same as the outer diameter of the sheath, for example, to provide a firm degree of reinforcement for the sheath.

In some embodiments, the projection is movable between a first portion not overlapping a predetermined region of the implant retainer and/or not overlapping the attachment element of the implant; and a second position overlapping at least partly the predetermined region of the implant retainer and/or overlapping at least partly the attachment element of the implant.

The expansion restrictor may be configured to translate axially with at least a portion of the translation of the sheath.

In some embodiments, the apparatus further comprising a transfer tube for constraining the implant in a compressed condition for loading into the delivery catheter, the transfer tube having a peripheral edge with a circumferential recess for keyed engagement with the projection.

Additionally or alternatively to any aspect above, and additionally or alternatively to any of the features described above, a closely related fourteenth aspect of the invention provides a method of loading a self-expanding cardiovascular implant into a delivery catheter, the delivery catheter comprising an accommodation region for accommodating the cardiovascular implant in a compressed configuration, and/or a sheath translatable longitudinally with respect to the accommodation region for selectively covering the implant to restraint the implant in the compressed configuration or selectively uncovering the implant to allow expansion for implantation, and/or an implant retainer in the accommodation region for mating engagement with at least one attachment element of the implant. The method of loading may comprise:
providing an expansion restrictor at the mouth of the sheath, at least a portion of the restrictor extending beyond the mouth of the sheath and including an abutment that is selectively movable with respect to the sheath by manipulation of the restrictor;
detachably engaging the attachment element of the implant with the implant retainer, while the sheath is at a first position not substantially covering the implant retainer;
manipulating the expansion restrictor such that the abutment is moved into a blocking position restraining the attachment element in engagement with the implant retainer; and
translating the sheath to at least partly close the sheath over the implant, the step of translating the sheath comprising directly or indirectly displacing at least the abutment of the expansion restrictor out of engagement with the attachment element as the sheath translates over the attachment element, such that the attachment element transitions from being restrained by the abutment to being restrained by the sheath, without detaching from the implant retainer.

In all of the above aspects, the invention may be defined independently of the implant, or in combination with the implant.

A further aspect of the invention provides a combination of a cardiovascular implant, and apparatus according to any aspect described herein, and optionally including any of the features described above. For example, the implant and the apparatus may be provided as a kit, and/or used together.

In any of the above aspects, optionally the implant has a non-round form and/or one or more out-of-round features.

Additionally or alternatively, in any of the above aspects, the implant has a plurality of protuberances, for example, projecting anchors. At least one protuberance may correspond to at least one interior longitudinal groove of the funnel. Optionally a plurality of protuberances may correspond to a plurality of interior longitudinal grooves of the funnel.

Certain exemplary embodiments of the present invention are described above, defined in appended claims and are illustrated in the accompanying figures. The embodiments described are only for purposes of illustrating the present invention and should not be interpreted as limiting the scope of the invention. Other embodiments of the invention and improvements of the described embodiments will become apparent with the following non-limiting description of embodiments. Protection is claimed for any novel feature or idea described herein and/or disclosed in the drawings, whether or not emphasis has been placed thereon.

### Brief Description of the Drawings

Fig. 1 is a schematic sectional view of a first embodiment in the form of compressor apparatus for compressing a cardiovascular implant, positioned in an initial state prior to compressing.
Fig. 2 is a schematic section view similar to Fig. 1, with the biasing device removed.
Fig. 3 is a schematic section view similar to Fig. 1, but with positioned in a partly compressing state.
Fig. 4 is a.schematic perspective view in isolation of a biasing device for the apparatus of Fig. 1.
Fig. 5 is a schematic side view of the apparatus of Fig. 2.
Fig. 6 is a schematic end view at the entrance of the apparatus of Fig. 2.
Fig. 7 is a schematic end view of the funnel of the apparatus in the state shown in Fig. 3.
Fig. 8 is a schematic top view of a prosthetic heart valve implant in its fully expanded configuration;
Fig. 9 is a schematic side view of the implant of Fig. 8;
Fig. 10 is a schematic top similar to Fig. 8 but in the compressed configuration of the implant;
Fig. 11 is a schematic side view similar to Fig. 9 but in the compressed configuration of the implant;
Fig. 12 is a schematic perspective view of a second embodiment in the form of an implant holder carrying an implant in a storage configuration;
Fig. 13 is a schematic underside perspective view of the implant holder carrying an implant in a storage configuration;
Fig. 14 is a schematic perspective view illustrating interaction between the compressor and the holder;
Fig. 15 is a schematic side view of a third embodiment in the form of a puller attached to an implant via a tether;
Fig. 16 is a schematic side view similar to Fig. 15, but showing the cover of the puller slightly removed from a hub;
Fig. 17 is a schematic perspective view of the hub of Fig. 16 in isolation;
Fig. 18 is a schematic representation of a path of return runs defined by a tether with respect to the hub of Figs. 16 and 17;
Fig. 19 is a schematic perspective view illustrating a fourth embodiment in the form of an expansion restrictor for assisting loading of an implant on a delivery catheter;
Fig. 20 is a schematic side section illustrating the expansion restrictor fitted around a sheath of a delivery catheter; and
Fig. 21 is an enlarged view showing detail of Fig. 20.

### Detailed Description of Embodiments

In the drawings, the same reference numerals are used to denote corresponding features whether or not described in detail.

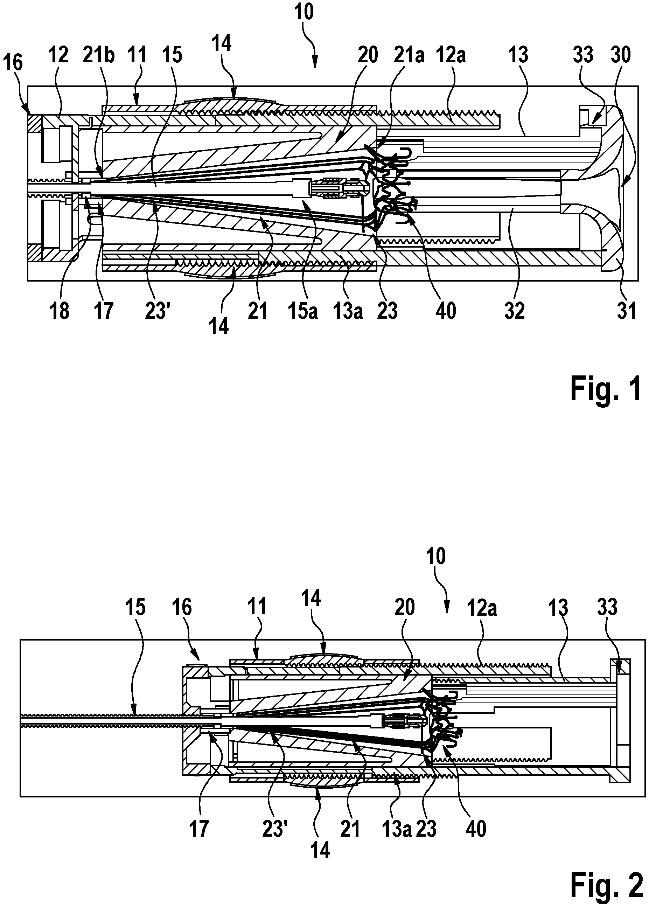

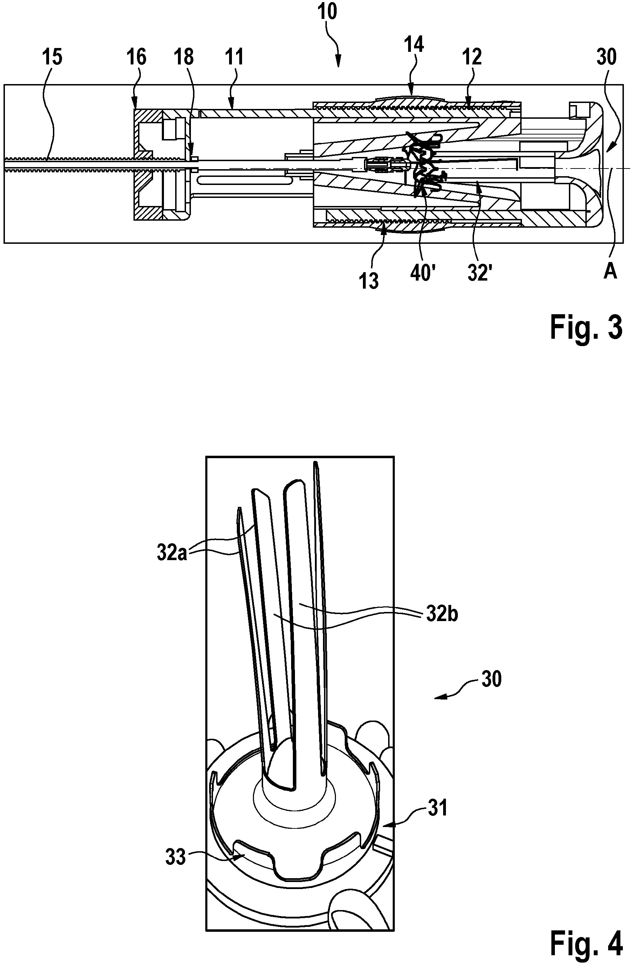

Figs. 1 to 7 illustrate an apparatus 10 for compressing a cardiovascular implant 40 to a compressed state 40' for transcatheter implantation. The apparatus may generally comprise or use at least one or any combination of: a funnel 20; a tapered channel 21 defined by the funnel 20; a channel 21 that changes in cross-section 21a and 21b; a biasing device 30; a mover 15 for pulling the implant 40 through the apparatus; an actuator mechanism 14 for driving movement of different parts of the apparatus.

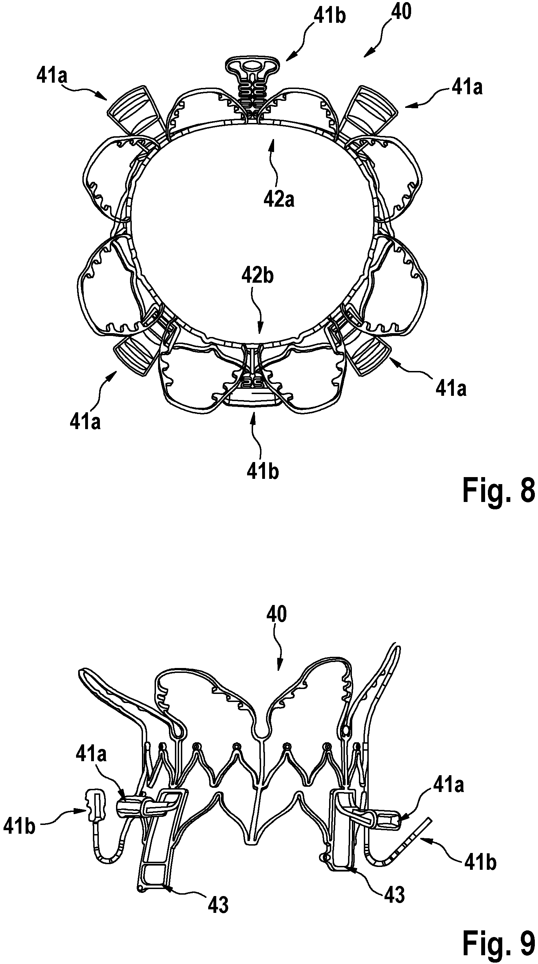

Referring to Figs. 8-11, the implant 40 is illustrated by way of example in the form of a self-expanding prosthetic mitral valve. Only the self-expanding frame of the implant is shown, to avoid obscuring the drawing. Figs. 8 and 9 depict the implant in its fully expanded configuration in which the implant is initially supplied, and towards which the implant will, in use, selfexpand upon implantation. In this expanded configuration, the implant 40 presents a non-circular profile. The implant has a generally D-shaped cross-section shape to fit a mitral valve anatomy. A flat or less-curved region 42a may correspond to an anterior region of the native mitral anatomy, and a more curved region 42b may correspond to a posterior region. The implant 40 further comprises an anchoring system comprising at least one protuberance 41, optionally plural protuberances 41, for example, six protuberances in the illustrated form. The protuberance(s) may serve to anchor the implant 40 with respect to native anatomical tissue. In the illustrated form, the protuberances include at least one side protuberance 41a (four in the illustrated example) and/or at least one end protuberance 41b (two in the illustrated example). In the expanded configuration of the implant, each side protuberance 41a extends outwardly from a window frame 43 formed in the tubular portion of the implant frame. Each end protuberance 41b is folded on itself to define a jaw-like anchor with respect to the tubular portion.

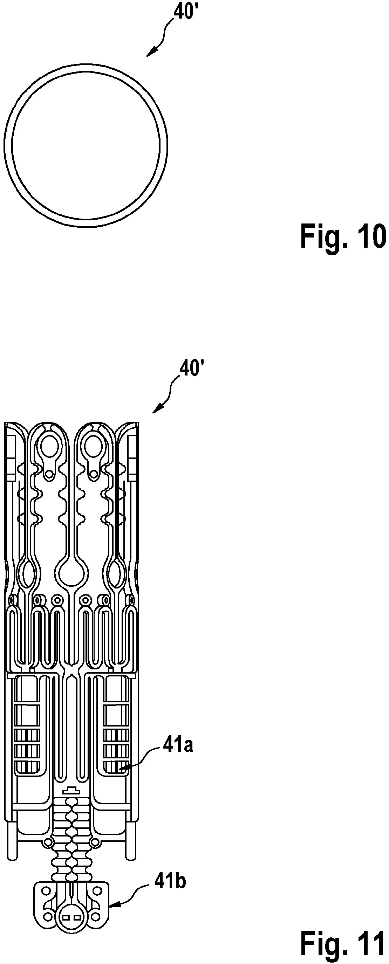

Figs. 10 and 11 depict the implant when compressed to a compressed configuration by the apparatus 10. The apparatus 10 compresses the implant from a non-circular profile to a generally round shape. The window frames 43 permit the side protuberances 41a to be folded inwardly to be coincident with the tubular portion. The window frame 43 is a generally non-compressible part of the frame that does not change significantly in dimension as the stent is compressed circumferentially. The frame may exhibit stress concentrations that are generally non-uniform around the periphery during compression, for example, due to its original non-circular profile and/or the presence of non-compressible windows or other frame cells having different resistances to circumferential compression. As best seen in Fig. 11, the end protuberances 41b are bendable through an angle of at least through at least 90°, optionally at least 120°, optionally at least 150°, optionally at least 170°, optionally about 180°, into a generally straight extension projecting beyond the end of the tubular portion.

The implant 40 further comprises a valve component (not shown in Figs. 8-11) carried by the frame, for forming a one-way flow regulating valve.

Referring back to Figs 1-7, the funnel 20 of the compressor apparatus 10 has an interior surface defining a tapered channel 21 for compressing the implant 40 from its initial expanded state to a compressed state 40' in response to longitudinal translation of the implant 40 within the channel 21. The channel 21 may have an axis A extending from an entrance 21a toward an exit 21b.

The channel 21 presents a change in cross-section size and/or shape, varying from or near an entrance 21a with a non-round or out-of-round cross-section 23 to or near an exit 21b with a smaller generally rounder cross-section 23'. The channel 21 may progressively change in cross-section so as to facilitate the change in size and shape of the implant in accordance with the longitudinal advancement of the implant within the channel. The channel 21 changing from a non-round or an out-of-round cross-section 23 to a generally round cross-section 23' permits the implant 40 to compress progressively and controllably from a non-round or an out-of-round shape to a generally round form that is compatible with transcatheter delivery in a patient.

In the illustrated example, the channel 21 has at its entrance a cross-section comprising a curved region connected to a lessercurved region; optionally the less-curved region may have a curvilinear region or a straight region. In other words, the channel 21 may provide a cross-section that may be likened to a D-Shape (optionally with one or more shape protuberances around the periphery, as discussed below).

Such a non-round or out-of-round profile of the channel 21, at least at the entrance, allows for the apparatus to be used in combination with cardiovascular implants of a similar profile, for example as illustrated in Figs. 8 and 9. A D-shaped implant can therefore be compressed with more control than using a purely round funnel channel not adapted to the implant shape.

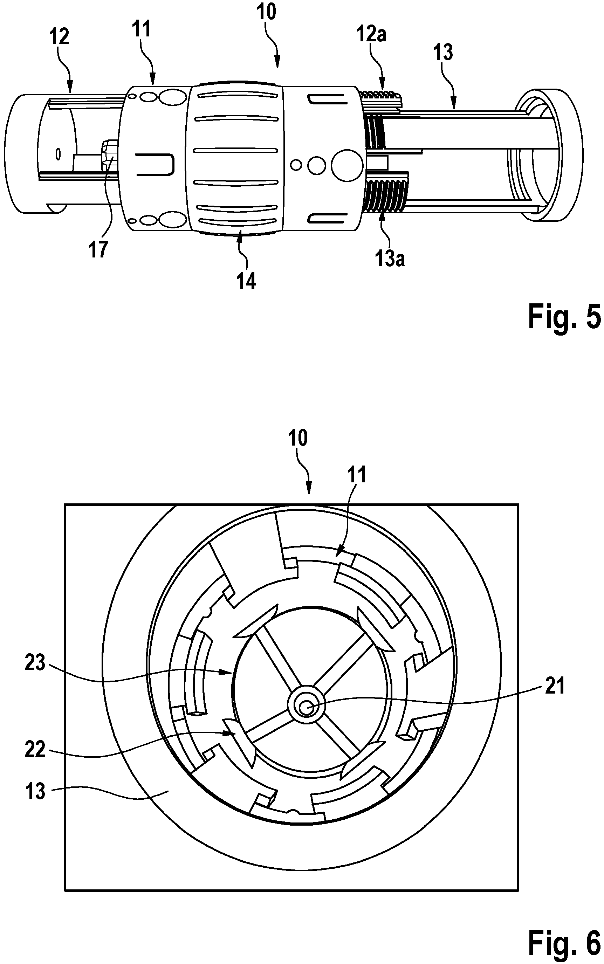

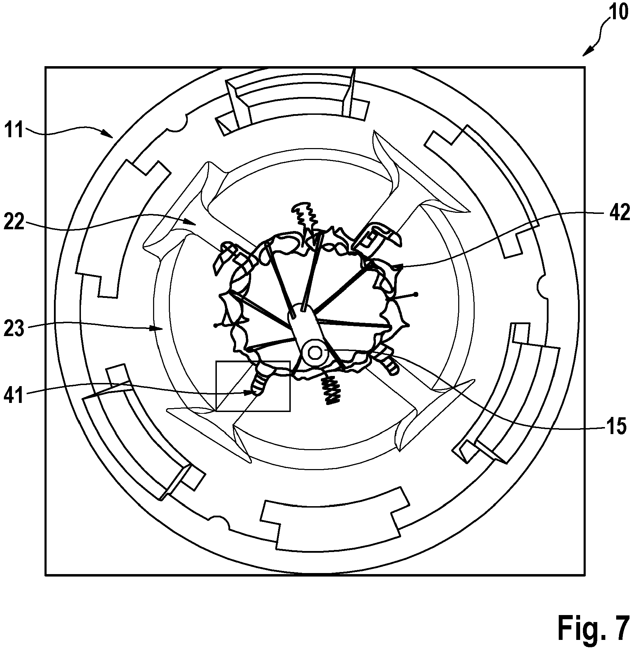

The interior surface of the funnel 20.also provides at least one longitudinal groove 22 (figs. 6 and 7), four such grooves 22 in the present example. The at least one groove 22 extends from the entrance 21a towards the exit 21b of the channel 21 and varies in depth as it extends longitudinally along the interior surface of the funnel 20. The groove 22 may become progressively shallower as it extends towards the channel exit. At the entrance of the channel, the grooves are visible as one or shape protuberances around the periphery.

Provision of the at least one groove 22 facilities compression of tubular implants comprising one or more external protuberances 41 (especially side protuberances 41a in this example). Referring to Fig. 7, the position of the or each groove 22 around the periphery is generally aligned with the position of a respective protuberance 41a of the implant 40 in order to receive the protuberance when the implant is introduced at the channel entrance. During compression, the groove 22 facilitates controlled inward folding of the protuberances against or into the implant wall, thereby reducing the risk of breakage or damage of the implant 40.

A biasing device 30 is also provided to bias at least one or more zones of the implant 40 outwardly, from within the implant 40, against at least a portion of the interior surface of the funnel 20, and hence reduce risk of inward kinking of the implant 40 during the compression phase. The biasing device 30 can support the one or more zones from within during the compression phase. The compression phase may correspond to any moment during which the implant 40 is longitudinally advanced within the channel 21. The one or more zones may optionally correspond to regions of the stent (e.g. the window frame regions) that do not compress substantially in the circumferential direction, and/or that support inwardly folding protuberances, and/or that have relatively high resistance to circumferential compression. Such zones may be vulnerable to inward kinking when the implant as a whole is forcibly compressed.

The biasing device 30 comprises one or a plurality of cantilevered biasing elements 32 extending longitudinally with respect of the channel 21, and inserted into the channel 21 in use via the entrance 21a to help keep at least a portion of the implant flush against the channel surface. At least two or more cantilevered elements 32 may have the same length as one-another 32a or may have different lengths from one another 32b. The or each cantilevered element 32 may be finger-like and extend longitudinally from a hub 31. Providing at least two or a plurality of cantilevered elements 32 helps to keep the implant 40 at least partially flush against the interior surface of the funnel 20 at multiple positions around its entire periphery, thus increasing the resistance to unwanted inward kinking.

The cantilevered element(s) 32 may be deflectable (e.g. capable of flexing) inwardly 32' so as to accommodate the change in profile of the channel 21 as the biasing device 30 advances longitudinally; optionally, the biasing device 30 may translated or be translated with the implant within the channel 21. The cantilevered element 32 may be deflectable or have a deflectable tip able to conform to the change in profile of the channel 21. Additionally or alternatively, the cantilevered element 32 may be biased outwardly 32' so as to urge the implant 40 against the interior surface of the funnel 20.

The biasing device 30 may further be configured and/or mounted so as to advance longitudinally with regards to the funnel 20, optionally the longitudinal movement of the biasing device 30 may be simultaneous or in unison with the advancement of the implant 40 within the channel 21. The advancing of the biasing device 30 within the channel 21 causes the cantilevered elements 32 to at least partially deflect inwardly whilst supporting the implant 40 and urging the implant 40 against at least a portion of the interior surface of the funnel 20. The cantilever element 32 may be configured to guide folding of the valve component of .the implant during compression. The valve component may be made of biological tissue or other biocompatible materials. Guiding the folding of the valve component may reduce risk of tearing, or permanent creasing or other damage that could be caused by random folding, and could otherwise reduce the working life of the valve component.

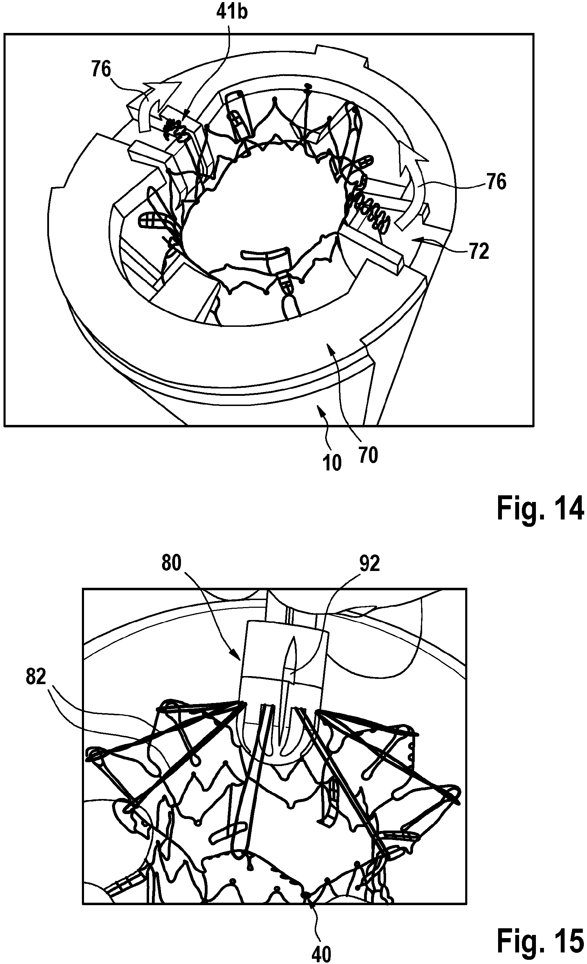

The apparatus 10 may further comprise a mover 15 for advancing the implant 40 longitudinally within the channel, by pulling the implant 40. The mover 15 comprises, for example, a threaded tube or shaft, having a tip structure 15a coupled to the flared end implant 40 by means of a puller 80 and one or more tethers 82 (e.g. Fig. 7). More detail of an embodiment of the puller 80 and the tethers 82 is described later with respect to Figs. 15 to 18. Pulling the mover 15 generates a force via the tethers to move the implant longitudinally while also encouraging the flared end to be drawn inwardly to a smaller circumference.

The apparatus 10 may further comprise an actuator mechanism 14 situated on a structure 11 coupled to or integral with the funnel 20, for driving longitudinal movement of a first element (e.g. the biasing device 30) from (or at or via) the entrance 21a of the channel 21a, and for generating longitudinal movement of a second element (e.g. the mover 15) from (or at or via) the exit 21b of the channel 21. The movement may be generated so as to produce simultaneous movement of the two elements, optionally in unison, over at least a portion of the range of movement of each element. The two elements may be translatable longitudinally with respect to the channel 21, and optionally at least partly with respect of each other.

The actuator mechanism 14 may comprise a manually rotatable member 14a operatively coupled to at least one carriage 12, 13 coupled in turn to the biasing device 30 and the mover 15. One or more screw threaded, or rack and pinion, or other couplings, converts manual rotation into longitudinal carriage movement. In the orientation shown in the drawings, the mover 15 moves to the left towards and out of the exit 21b, while the biasing device 30 also moves to the left from the entrance end 21a of the channel 21. In the illustrated embodiment, two carriages 12, 13 are used to enable more refined control of the movement of each. A first carriage 13 includes a first connector ring 13a to which the hub 31 of the biasing device 30 can be attached, for example, by a snap-fit connection 33. A second carriage 12 includes a second connector ring 12a carrying a rotatable adjuster 16 to which the mover 15 is threadedly coupled. The adjuster 16 permits additional adjustment of the longitudinal position of the mover 15, or additional pulling of the mover 15, in response to rotation of the adjuster 16.

The actuator mechanism 14 thus facilitates operation by a single person, enabling coordinated movements of both the mover 15 and the biasing device 30, which can be advantageous for the aim of optimal compression of the implant. Facilitating operation by a single user contributes to a significantly less labour intensive compressing procedure.

The apparatus 10 further provides a transfer tube 17 removably positioned at the exit end 21b of the channel 21, for receiving the compressed implant 40' as it emerges from the channel 21 in its compressed state. The transfer tube 17 can retain the implant 40 in its compressed form for transfer to the delivery catheter for loading.

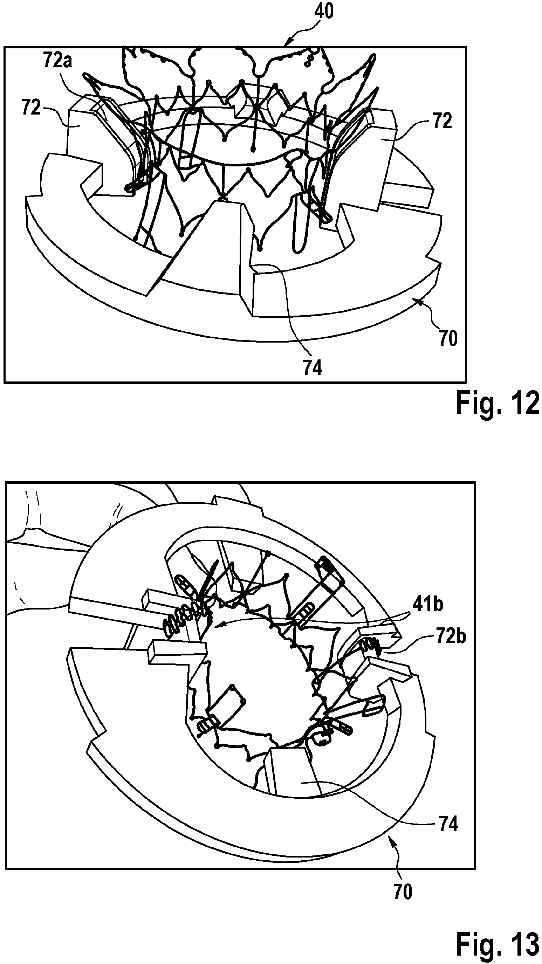

Figs. 12-14 illustrate a second embodiment relating to a multifunctional implant holder 70 for supporting the implant 40 during storage, and for assisting manipulation of the implant 40 as it is transferred to apparatus for compressing the implant. Figs. 15-18 illustrate a third embodiment relating to a puller 80 coupled to the implant 40 via one or more tethers 82. The second and third embodiments are illustrated separately, but described below together, as these embodiments may be used independently or in combination. The second and/or third embodiment may optionally be used with the first embodiment above.

Referring to Figs. 12-14, prior to use, the implant 14 is stored in its fully (or near fully) expanded configuration, for example in sterile packaging such as a bottle (not shown). The holder 70 is generally ring shaped and at least partly circumscribes the implant 14 sitting at least partly within the holder 70.

The holder includes at least one engagement profile 72 (two profiles 72 in the illustrated embodiment) aligned with a respective end protuberance 41b, and around which the protuberances 41b engage to retain the implant 40 securely coupled to the holder 70. Each engagement profile 72 includes a contoured front face 72a providing a form-fit with the implant 40, and a channel-shaped rear profile .72b for accommodating the end protuberance 41b. The holder 70 further optionally further comprises one or more side rests 74 for stabilising the implant 40 in the holder 70.

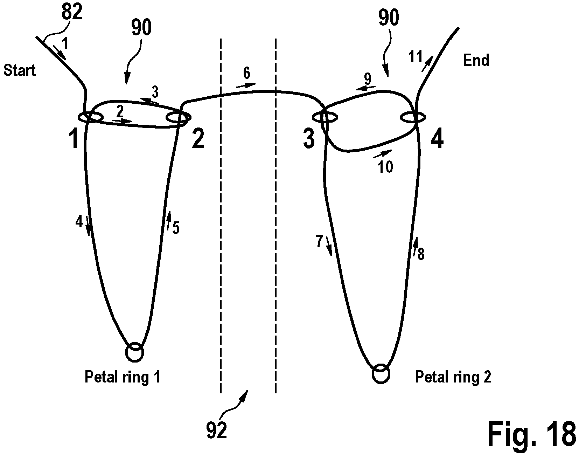

Referring to Figs. 15-18, also prior to use, the implant 14 is provided pre-attached to a puller 80 via at least one tether 82 defining multiple return runs between the puller 80 and apertures of the implant 40, for example, apex apertures of a flared atrial end of a heart-valve stent. The puller 80 comprises a hub 84 and an optional cover 86 mountable to the hub 84. The hub 84 comprises a plurality of apertures 88 through which the tether 82 extends. In the illustrated example, the apertures 88 have the form of axial bores. The apertures 88 are provided in a circumferential arrangement around a periphery of the hub 84. In the schematic view of Fig. 18, the axial bores are collapsed as simple schematic apertures.

Referring to Fig. 18, the tether 82 is threaded in a predetermined manner to resist or obstruct lengthwise slippage of the tether 82 with respect to the puller 80. During compression of the implant 40, the puller is used apply traction to one end of the implant 40 to pull the implant 40 into and/or through a compressor apparatus. Avoiding slippage of the tether 82 with respect to the bores 88 can maintain good control of alignment of the implant 40 with the axis of the puller, and ensure uniform application of the traction forces around the periphery of the implant 40. Such characteristics are beneficial to a controlled compression of the implant, especially in the case of a non-round configuration or protruding anchors.

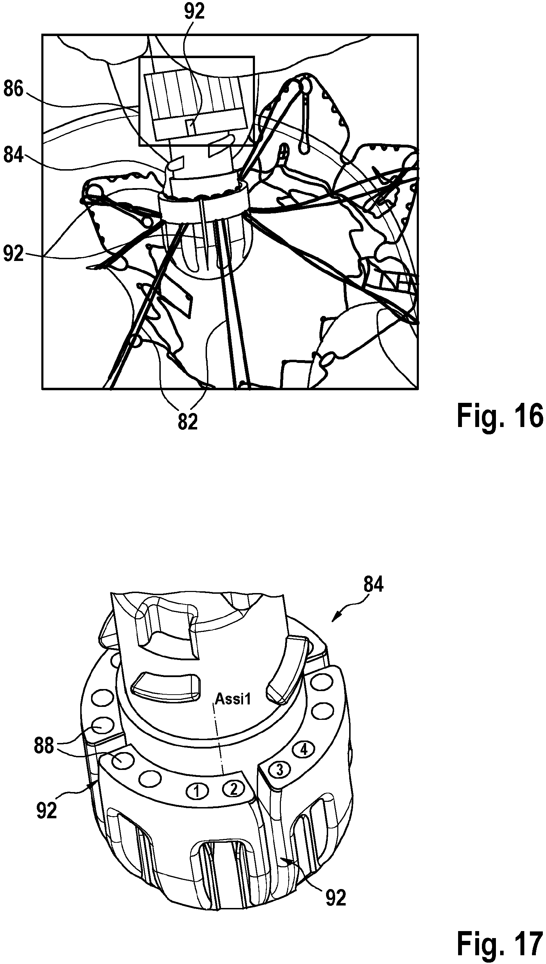

Lengthwise slippage of the tether 82 is obstructed by.the path of the tether 82 including at least one, optionally plural, anchor loops 90 with respect to certain apertures. Fig. 18 depicts a path of the tether, in a sequential order of path segments indicated by arrows 1-11, from "start" to "end", defined with respect to four apertures 88 (labelled 1-4), also identified in Fig. 17. The tether 82 extends from above (Figs. 17 and 18) through a first aperture 1, along an underside path segment 2 to the second aperture 2, passes through the aperture 2, and returns to the first aperture 1 along an upperside third path segment 3. The tether 82 passes again through the first aperture 1, and extends on a run 4 out to an apex of the implant, and back to the puller on run 5, passing again through the second aperture 2. The runs 4 and 5 define a return run between the puller 80 and the implant 40. The path segments 2 and 3 define an anchor loop 90 by tightly wrapping between the first and second apertures 1 and 2 to resist lengthwise slippage. After passing through the second aperture 2, the tether extends towards the next pair of apertures 3 and 4, to repeat the same path sequence.

If' desired, lengthwise slippage may also be resisted by clamping contact with the cover 86, although in other embodiments, the cover 86 may serve to only to shroud and protect the tether 82 (as described below) without clamping contact.

The puller 80 further comprises one or more, in this embodiment four, cutting gaps 92 at which the tether 82 is presented, to facilitate cutting the tether at precise locations when it is desired to release the puller 80 from the implant (e.g. after crimping). The gaps 92 are provided in the hub 84 in the form of axial grooves, and in the cover 86 in the form of axial slots that align with the grooves in the hub 84 when the cover 86 is assembled to the hub 84. The cutting gap 92 is dimensioned to enable the tether 82 to be cut within the gap 92 by inserting the edge of a scalpel into the gap 92. The cover 86 protects the tether 82 against accidental cutting damage either side of the intended cutting gaps 92.

As can be seen in Fig. 18, the tether 82 includes a path segment 6 that extends across the cutting gap 92 between adjacent pairs of apertures 88. When the tether 82 is cut at path segment 6, the tether separates into different tether segments. The path segments 5 and 7 are free to pull out from the bub 84, allowing release of the implant 40. The tether 82 remains attached to the hub 84 because the anchoring loops 90 (path segments 2 and 3; and path segments 9 and 10) prevent the tether 82 from easily slipping out at these end positions.

The at least one tether 82 may comprise a single tether defining all of the multiple runs between the puller 80 and the implant 40, or multiple tethers may be used, for example, each extending around a respective sector of the periphery of the puller and/or implant. In the illustrated embodiment, sixteen apertures 88 support eight return runs of the tether 82 between the puller 80 and the implant 40. The apertures are grouped into fours. Four cutting gaps 92 are arranged between adjacent groups of apertures 92. Four tethers 82 may optionally be used, or two tethers, or a single tether.

When it is desired to compress the implant 40 for loading into a delivery catheter, the implant 40 is transferred to the compressor apparatus (e.g. the apparatus 10 of the first embodiment) optionally using the holder 70. For example, referring to Fig. 14, the holder 70 is introduced at an insertion position, e.g. at the entrance end 21a, with respect to the channel 21, with the end protuberances 41b facing away from the apparatus 10.

The mover 15 is coupled to the end of the implant closest to the channel 21 for pulling the implant 40 into the channel 21. For example, the mover 15 is coupled to the puller 80 described above.

As the implant 40 is pulled and translates out of the holder 70 and into the channel 21 (e.g. downwardly in Fig. 14), the engagement profiles 72 cooperate with the end protuberances 41b, causing the end protuberances 41 to bend (as depicted by arrows 76) towards their non-deployed configuration in which the protuberances 41b form a straight extension pointing away from the remainder of the implant 40. Using the holder 70 to automatically bend the protuberances 41b to straight (or nearly straight) means that this operation does not need to be performed separately by the compressing funnel, and does not complicate engagement between the implant 40 and the funnel 21. The engagement profile(s) 72 may bend the protuberances through at least 90°, optionally at least 120°, optionally at least 150°, optionally at least 170°, optionally about 180°.

The biasing device 30 may be introduced into the interior of the implant 40 before translating out of the holder, or after once the implant 40 is substantially within the channel 21.

Once the implant 40 has been loaded into the transfer tube 17, the puller 80 may be released from the implant 40 by cutting the tether 82 at the cutting gaps 92 of the puller 80. One run of each return run becomes freed, while the other run retains the tether captive to the puller 80, avoiding any tether debris remaining attached to the implant 40.

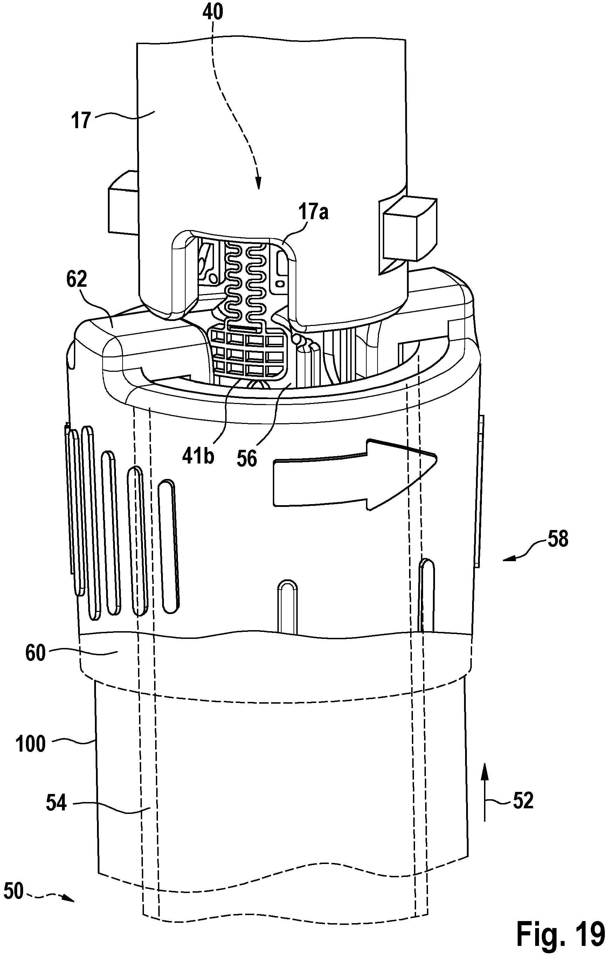

Figs. 19 to 21 illustrate a fourth embodiment relating to an expansion restrictor 58 for assisting loading of the compressed implant 40 in a delivery catheter 50. The fourth embodiment may optionally be useable with the first and/or second and/or third embodiments described above, but especially with the first embodiment.

Referring to Fig. 19, the transfer tube 17 is shown being used to transfer the compressed implant 40 from the compressor apparatus 10 to an implant accommodation region of a delivery catheter 50. The delivery catheter includes a sheath 54 translatable longitudinally with respect to the accommodation region for selectively covering the implant 40 to restraint the implant 40 in the compressed configuration, or selectively uncovering the implant 40 to allow expansion for implantation.

An implant retainer 56 in the accommodation region releasably engages at least one attachment element of the implant 40, to axially restrain the implant in its desired position. In the present example, the attachment element is provided by one or both end protuberances 41b when extended axially in the compressed configuration. The implant retainer 56 has a profile to receive and matingly engage the tip (a paddle shaped end profile) of the protuberance(s) 41b.

Referring generally to Figs. 19 - 21, the expansion restrictor 58 is provided on or is positionable on the catheter to assist with loading of the implant on the delivery catheter. The expansion restrictor 58 comprises a tubular member 100 having a bore dimensioned to receive the sheath 54 while providing a snug fit around the exterior of the sheath 54. The tubular member 100 reinforces the sheath 54 to provide additional column strength during the loading process, and reduce risk of sheath buckling when the sheath is translated to close over the implant.

The expansion restrictor 58 further comprises a manipulator ring 60 at the mouth of the tubular member, from which extends at least one projection 62, two in the illustrated example. The (or each) projection 62 extends radially inwardly to a position that is substantially in register with the inner surface if the sheath 54, in order to provide a continuum of constraint on the implant between the sheath 54 and the projection 62. The (or each) projection 62 also extends axially proud of the ring 60, to define a circumferential castellation. The ring 60 is rotatable, or at least movable circumferentially, around the axis of the tubular member 100, to move the projection 62 between a blocking position with respect to the attachment element and/or the retainer (rotated anticlockwise in the direction of the arrow in Fig. 19), and a non-blocking position (rotated clockwise in Fig. 19).

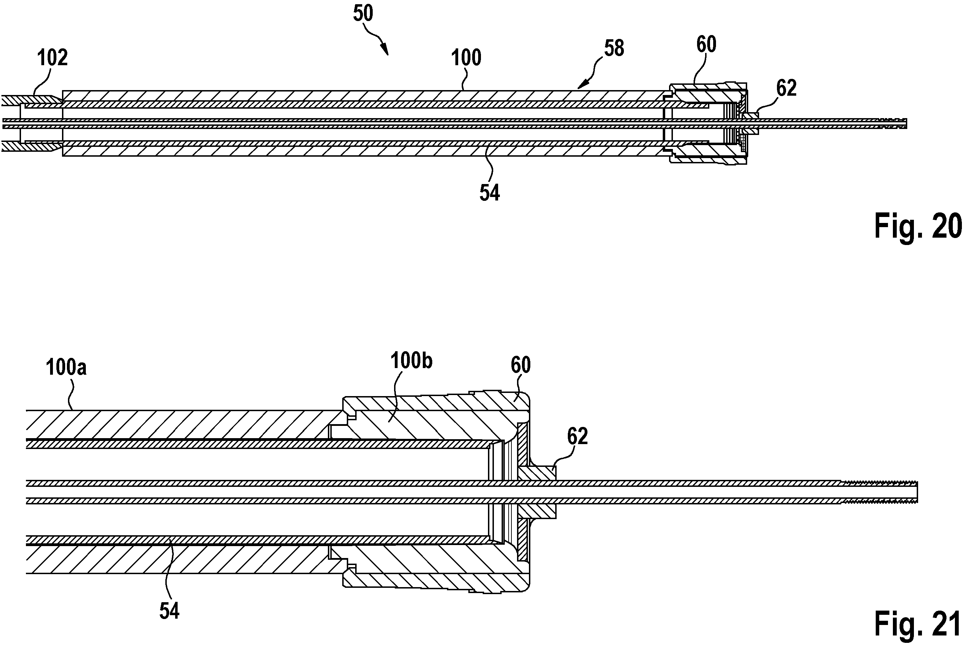

As best seen in Fig. 21, in the present example, the tubular member 100 may be made in two parts attached together, for example, a main tubular body part 100a, and a mouth part 100b shaped and dimensioned as a support hub around which the manipulator ring 60 is mounted. Such a construction may also facilitate assembly of the ring 60 to the tubular member 100 in a captive, rotatable manner.

In use, if not already installed on the catheter 50, the expansion restrictor 58 is slid over the sheath 54 of the delivery catheter to prepare for loading of the implant. Following compression of the implant (e.g. using the apparatus 10 described above), the implant 40 is transferred to the delivery catheter 50 using the transfer tube 17. The transfer tube 17 is slightly shorter than the implant 40, or has end recesses 17a, exposing the attachment elements (the tips of the end protuberances 41b). With the ring 60 set in the non-blocking position, and the sheath in the open state, the attachment elements can be manually engaged with the implant retainer 56. The person performing this task may need to slightly squeeze the attachment elements radially inwardly to counter the natural self-expansion tendency of the implant 40. In the non-blocking position, the expansion restrictor 58 allows easy finger access and visual access to correctly engage the attachment elements with the retainer 56.

Thereafter, the ring 60 is manually rotated to move the projection(s) 62 circumferentially into its blocking position (in the direction of the arrow in Fig. 19) in which the projection 62 overlaps at least partly the attachment element and/or the engagement profile of the retainer 56. The projection 62 overcomes the self-expanding bias of the implant at the attachment element, and blocks the attachment element in operative engagement with the implant retainer 56. This provides stable and reliable engagement without further interaction by the user, freeing the user's hands for other tasks and/or avoiding the need for an additional operator to be present.

The delivery catheter 50 is operated to translate the sheath 54 closed in the direction of sheath arrow 52, transitioning the attachment element from being restrained by the projection 62, to being restrained by the advancing sheath 54, and without detaching from the implant retainer 56. Such a technique can simplify the loading operation, and facilitate advantageous loading of the delivery catheter by a single person. As the sheath 54 is translated closed, the tubular member 100 provides support and reinforcement around the sheath to prevent bucking under column loads at the sheath 54. Also as the sheath 54 is translated closed, the expansion restrictor 58 can be progressively displaced, e.g. directly by the advancing sheath and/or indirectly via contact between an advancing portion 102 of the delivery catheter that moves the sheath 54. Axial displacement of the expansion restrictor 58 moves the projection 62 away from overlapping the implant retainer 56 and/or the attachment element of the implant. In turn, the displacement of the restrictor 58 also displaces the transfer tube 17, so that the implant 40 transitions progressively from being restrained by the transfer tube 17, to being restrained by the sheath, without further manual intervention of the user at the mouth of the sheath 54. Thereafter, the expansion restrictor 58, especially the tubular member 100 remaining around the sheath 54, can be removed by sliding off the sheath, optionally dislodging at the same time the transfer tube 17 if still engaged with the delivery catheter.

It will be appreciated that the foregoing description is merely illustrative of preferred embodiments of the invention, and that many modifications and equivalents may be used within the scope and/or principles of the invention.

## Claims

1. Apparatus (10) for compressing a cardiovascular implant (40), optionally a prosthetic heart valve, to a smaller size for transcatheter implantation, comprising:
a funnel (20) having an interior surface defining a tapered channel (21) configured for compressing the implant in response to longitudinal translation of the implant within the channel; and
a device (30) for biasing the implant (40) outwardly against at least a portion of the interior surface of the funnel as the implant translates longitudinally within the channel,
optionally to prevent inward kinking during compression of an implant having a noncircular profile.

2. Apparatus according to claim 1, wherein the biasing device (30) comprises at least one cantilevered biasing element (32) extending generally longitudinally with respect to the channel (21), and configured to be (i) biased outwardly towards at least a portion of the interior surface of the funnel, and/or (ii) deflectable inwardly to accommodate insertion into the tapered channel.

3. Apparatus according to claim 2 wherein the biasing device (30) comprises at least two cantilevered biasing elements 832; 32a, 32b), said elements having the same and/or different lengths.

4. Apparatus (10) for compressing a cardiovascular implant (40), optionally a prosthetic heart valve, to a small size for transcatheter implantation, the apparatus optionally according to claim 1, 2 or 3, and the apparatus comprising:
a funnel (20) having an interior surface defining a tapered channel (21) configured for compressing the implant in response to longitudinal translation of the implant within the channel, the channel having an axis (A) extending from an entrance (21a) to an exit (21b); and
at least one, optionally a plurality, of longitudinal cantilevered elements (32; 32a, 32b) configured for insertion into the channel via the entrance for supporting the implant (40) from within during compression, the at least one cantilever element having a tip biased towards a position spaced from the axis of the channel, the tip being deflectable towards the axis of the channel to accommodate tapering of the channel.

5. Apparatus according to claim 3 or 4, wherein the cantilevered elements (32; 32a, 32b) are fingers extending from a hub (31).

6. Apparatus according to any preceding claim, wherein the apparatus (10) is configured for compressing to a round form an implant having a noncircular profile.

7. Apparatus according to any preceding claim further comprising an actuator mechanism (74) for generating longitudinal movement of the biasing device (30) and/or cantilevered element (32; 32a, 32b) with respect to the funnel (20), optionally wherein the actuator mechanism comprises a screw threaded element for generating longitudinal movement from rotation of a rotatable actuator member.

8. Apparatus according to claim 7, further comprising a mover (15) for advancing the implant within the channel, the mover (15) being driven by the actuator mechanism (74).

9. Apparatus according to claim 8, wherein the actuator mechanism (74) is configured to cause simultaneous longitudinal movement of the biasing device (30) and the mover (15) over at least a part of the range of movement of the biasing device and/or of the mover.

10. Apparatus according to claim 9, wherein the actuator mechanism (74) is configured to cause longitudinal displacement of the biasing device (30) in unison with longitudinal displacement of the mover (15) over at least a part of the range of movement of the biasing device and/or of the mover.

11. Apparatus according to any preceding claim, wherein the tapered channel (21) has a cross-section that changes in size and shape from one end (21a) of the funnel to the other (21b), the shape changing between a non-round channel shape and a round channel shape, for compressing to a round form an implant having a noncircular profile.

12. A combination of apparatus according to any preceding claim, and a cardiovascular implant (40), optionally wherein the cardiovascular implant is a prosthetic heart valve.

13. A method of compressing a cardiovascular implant (40), optionally a prosthetic heart valve, to a smaller size for transcatheter delivery, the method optionally using apparatus according to any of claims 1 to 11, the method comprising:
translating the implant (40) longitudinally within a funnel (20) having an interior surface defining a tapered channel (21), such that the tapered channel compresses the implant in response to the longitudinal translation; and
during at least a part of the translation step, supporting .at least one zone of the implant from radially within to resist kinking of said zone during compression.

14. A method of compressing a cardiovascular implant (40) to a smaller size for transcatheter delivery, the method optionally according to claim 13, and/or the method optionally using apparatus according to any of claims 1 to 11, the method comprising:
translating the implant (40) longitudinally within a funnel (20) having an interior surface defining a tapered channel (21), such that the tapered channel compresses the implant in response to the longitudinal translation; and
during at least a part of the translation step, supporting at least one zone of the implant from radially within by at least one, optionally a plurality of, longitudinal cantilevered elements (32; 32a, 32b).

15. The method of claim 13 or 14, further comprising translating the biasing device (30) and/or the cantilevered element (32; 32a, 32b) with respect to the funnel (20).

16. A combination according to claim 12, or a method according to any of claims 13 to 15, wherein the cardiovascular implant (40) has a non-circular profile in its non-compressed state.
